# EUROPEAN PATENT SPECIFICATION

(11) **EP 4 320 097 B1**
(45) Date of publication and mention of the grant of the patent: **13.11.2024**
(21) Application number: 22761158.9
(22) Date of filing: 04.08.2022
(51) Int. Cl.: C07C 323/22, G03F 7/00, C08F 2/46, G03F 7/027, G03F 7/031

(54) **NOVEL PHOTOINITIATOR**
NEUER PHOTOINITIATOR
NOUVEAU PHOTO-INITIATEUR

(30) Priority: 08.10.2021 IT 202100025868
(43) Date of publication of application: 14.02.2024
(73) Proprietor: IGM Resins Italia S.r.l., 20154 Milano (IT)
(72) Inventor: MORONE, Marika, 20154 Milano (IT); RAZZANO, Vincenzo, 20060 Bussero (IT); VAN DEN BRANDEN, Stefan Jean Yves Marie, 20123 MIlano (BE)
(74) Representative: Gislon, Gabriele
(86) International application number: PCT/EP2022/071955
(87) International publication number: WO 2022/238592

(56) References cited:
- EP-A1- 3 597 668
- EP-A1- 3 597 669
- CN-A- 112 441 953
- KR-A- 20210 066 736

## Description

### ABSTRACT

The present invention relates to novel photoinitiator having improved reactivity and surface curing and/or lower post-cure yellowing and to its use in photopolymerization compositions. The invention also concerns a process for the photopolymerization of compositions comprising said photoinitiator as well as their use in articles of manufacture, including printed, coated, and fabricated assemblies.

### PRIOR ART

In the last years, the design and the development of new photoinitiators (PIs) has been attracted increasing attention due to the large amount of photoinitiators that have been or are going to be banned as toxic or reprotoxic.

Various attempts were made to develop new photoinitiators able to mimic standard photoinitiators or to overcome problems such as yellowing, higher line speed, curing under LED lamps, some examples are glyoxylate 3-ketocoumarins (WO2021070152), Benzoyl phenyltelluride PIs (Macromolecules, 2014, 47(16), 5526-5531), silicon based PIs (JP2010229169, Macromolecules, 2009, 42(16), 6031-6037, Macromolecules 2007, 40(24), 8527-8530, Macromol. Rapid Commun. 2017, 38, 1600470, Macromolecules, 2017, 50(17), 6911-6923), fluorine based PIs (US2019/0155153). Further photo-initiators are known from EP3597669, EP3597668, KR20210066736 and CN1 12441953.

Unfortunately, when used in standard applications, also these photoinitiators show some limitations, such as low surface curing and, most importantly, post-cure yellowing, which makes said PI inappropriate in polymerizable systems involving transparent varnishes.

So, there exists the need of new technical solutions able to improve the surface curing of PIs without affecting the good reactivity of these products and/or to limit post-cure yellowing.

### AIMS OF THE INVENTION

It is a first aim of the invention to provide for a novel PI, its use as a photoinitiator and photocurable compositions comprising them.

It is a further aim of the invention to provide for photocurable compositions comprising the novel PI of the invention.

Is a further aim of the invention to provide for processes for photocuring ethylenically unsaturated compounds using the novel PI of the invention, as well as articles of manufacture made by said process.

### DESCRIPTION OF THE INVENTION

Surprisingly, we found that one photoinitiatior well reacts to UVA, UVB and UVC wavelength ranges as well as, and more preferably reacts to LED sources emitting in the range from 350 to 420 nm, maintaining a low post cure yellowing representing a technical progress compared to prior art.

It was unexpectedly found that one specific photoinitiator shows excellent performances, even better than those of two known PIs having very similar structures.

According to one of its aspects, the present invention relates to a compound of formula (I)

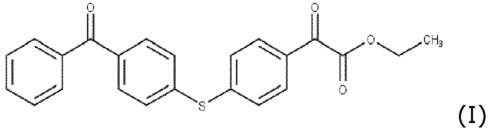

The compound of the invention can be prepared according to any suitable process. For example, it can be prepared by double Friedel-Crafts acylation.

According to another of its aspects, the present invention relates to a process for the preparation of the compound of formula (I) according to Example 1 below.

According to another of its aspects, the present invention relates to a photopolymerizable composition comprising:
a) from 50 to 99.9%, preferably from 70 to 98.9% by weight, based on the total content of the composition, of at least one ethylenically unsaturated compound;
b) from 0.1 to 35%, preferably from 0.1 to 20%, and more preferably from 0.2 to 15% by weight, based on the total content of the composition, of the compound of formula (I) as above defined; and
c) from 0 to 20% by weight, preferably from 0 to 15%, and more preferably from 0.2 to 15% by weight, based on the total content
of the composition, of an accelerator and/or of a coinitiator.

According to the present invention, the terms "photocuring" and "photopolymerizing" and related terms, are synonyms.

The expression "based on the total content of the composition" means that the % weight amounts of any of the components is calculated with respect to the sum of the weight of all the components of the composition, including any possible further additional components (in addition to a), b) and c) above), but possible water and/or solvents which may be present in the composition are not considered for the calculation of said % weight amounts.

The photocurable compositions of the present invention can also comprise one or more of the following components: (d) photosensitizers and/or (e) further photoinitiators and/or (f) conventional additives, in addition to compounds (a), (b) and, when present, (c).

According to another of its aspects, the present invention relates to a process for photocuring photopolymerizable compositions coatings, adhesives and inks, which process comprises:
i. providing a photopolymerizable composition as above defined;
ii. coating or printing said photopolymerizable composition onto a substrate, and
iii. photocuring said coated or printed composition with a light source on said substrate.

According to another of its aspects, the present invention relates to a process for three-dimensional printing which comprising photocuring with a light source a mixture comprising the composition as above defined.

Also described, but not part of the claimed invention, is an article of manufacture obtained by the process of the invention. According to a preferred embodiment, the photopolymerizable composition used the processes of the invention comprises at least components (a), (b), (c), more preferably at least (a), (b), (c), and (d) as above defined. The photoinitiators of the invention can be used in photocurable compositions comprising ethylenically unsaturated compounds (a). Said unsaturated compounds (a) can contain one or more olefinic double bonds. They can be low-molecular weight (monomeric) or high-molecular weight (oligomeric) compounds.

Examples of suitable low molecular weight monomers (monomeric compounds) having one double bond are alkyl or hydroxyalkyl acrylates or methacrylates, such as methyl-, ethyl-, butyl-, 2-ethylhexyl-,2-hydroxyethyl- or isobornyl-acrylate; and methyl or ethyl methacrylate. Further examples are resins modified with silicon or fluorine, e.g. silicone acrylates. Further examples of these monomers are acrylonitrile, acrylamide, methacrylamide, N-substituted (meth)acrylamides, styrene, alkylstyrenes and halogeno styrenes, vinyl esters such as vinyl acetate, vinyl ethers such as iso-butyl vinyl ether, N-vinylpyrrolidone, vinyl chloride or vinylidene chloride.

Examples of monomers having more than one double bond are the ethylene glycol diacrylate, propylene glycol diacrylate, neopentyl glycol diacrylate, hexamethylene glycol diacrylate, bisphenol A diacrylate, 4,4'-bis-(2-acryloyloxyethoxy)-diphenylpropane, trimethylolpropane triacrylate, pentaerythritol triacrylate or tetraacrylate, vinyl acrylate, divinyl benzene, divinyl succinate, diallyl phthalate, triallyl phosphate, triallyl isocyanurate or tris-(2-acryloylethyl) isocyanurate.

Examples of high-molecular weight (oligomeric) polyunsaturated compounds are acrylated epoxy resins, acrylated or vinyl-ether- or epoxy-group-containing polyesters, acrylated polyurethanes or acrylated polyethers. Further examples of unsaturated oligomers are unsaturated polyester resins which are usually prepared from maleic acid, phthalic acid and one or more diols and which have molecular weights of from about 500 Da to 3,000 Da. Such unsaturated oligomers can also be referred to as prepolymers.

Examples of compounds (a) which are particularly suitable for the implementation of the present invention, are esters of ethylenically unsaturated carboxylic acids and polyols or polyepoxides, and polymers containing ethylenically unsaturated groups in the chain or in side groups, e.g. unsaturated polyesters, polyamides and polyurethanes and copolymers thereof, alkyl resins, polybutadiene and butadiene copolymers, polyisoprene and isoprene copolymers, polymers and copolymers having (meth)acrylic groups in side chains, as well as mixtures thereof. Illustrative examples of unsaturated carboxylic acids or anhydrides, useful for the preparation of the above esters, are acrylic acid, methacrylic acid, maleic anhydride, crotonic acid, itaconic acid, cinnamic acid and unsaturated fatty acids such as linolenic acid and oleic acid. Acrylic and methacrylic acid are preferred.

Examples of polyols, which can also be esterified, are aromatic and aliphatic and cycloaliphatic polyols, preferably aliphatic and cycloaliphatic polyols.

Aromatic polyols are, for example, hydroquinone, 4,4'-dihydroxydiphenyl, 2,2-di(4-hydroxyphenyl) propane, as well as novolaks and resoles. Polyepoxides, which can be esterified, include those based on the said polyols, especially the reaction products between aromatic polyols and epichlorohydrin. Also suitable as polyols are polymers and copolymers that contain hydroxyl groups in the polymer chain or in side groups, for example polyvinyl alcohol and copolymers thereof or polymethacrylic acid hydroxyalkyl esters or copolymers thereof. Further suitable polyols are oligoesters carrying hydroxyl terminal groups.

Examples of aliphatic and cycloaliphatic polyols include alkylenediols containing preferably from 2 to 12 carbon atoms, such as ethylene glycol, 1,2- or 1,3-propanediol, 1,2-, 1,3- or 1,4-butanediol, pentanediol, hexanediol, octanediol, dodecanediol, diethylene glycol, triethylene glycol, polyethylene glycols having molecular weights of preferably from 200 Da to 1,500 Da, 1,3-cyclopentanediol, 1,2-, 1,3- or 1,4-cyclohexanediol, 1,4-dihydroxymethyl cyclohexane, glycerol, tris(β-hydroxy-ethyl)amine, trimethylolethane, trimethylolpropane, pentaerythritol, dipentaerythritol and sorbitol.

Further suitable ethylenically unsaturated compounds (a) are unsaturated polyamides obtained from unsaturated carboxylic acids and aromatic, aliphatic and cycloaliphatic polyamines having preferably from 2 to 6, preferably from 2 to 4, amino groups. Examples of such polyamines are: ethylenediamine, 1,2- or 1,3-propylenediamine, 1,2-, 1,3- or 1,4-butylenediamine, 1,5-pentylenediamine, 1,6-hexylenediamine, octylenediamine, dodecylene diamine, 1,4-diaminocyclohexane, isophoronediamine, phenylene diamine, bisphenylenediamine, di-(β-aminoethyl) ether, diethylene triamine, triethylenetetramine and di(β-aminoethoxy)- and di(β-aminopropoxy)ethane. Other suitable polyamines are polymers and copolymers which may contain additional amino groups in the side chain and oligoamides containing amino end groups.

Specific examples of such unsaturated polyamides are methylenebisacrylamide, 1,6-hexamethylene bisacrylamide, diethylenetriamine trismethacrylamide, bis(methacrylamidopropoxy) ethane and N-[(β-hydroxyethoxy)ethyl]-acrylamide.

Unsaturated polyurethanes are also suitable for the implementation of the present invention as components (a), for example those derived from saturated or unsaturated diisocyanates and unsaturated or saturated diols. Polybutadiene and polyisoprene and copolymers thereof may also be used. Suitable monomers include, for example, olefins, such as ethylene, propene, butene and hexene, (meth)acrylates, acrylonitrile, styrene and vinyl chloride.

Polymers having unsaturated (meth)acrylate groups in the side chain can also be used as component (a). They may typically be reaction products of epoxy resins based on novolac with (meth)acrylic acid; homo- or copolymers of vinyl alcohol or hydroxyalkyl derivatives thereof that have been esterified with (meth)acrylic acid; and homo- and co-polymers of (meth)acrylates that have been esterified with hydroxyalkyl (meth)acrylates.

According to a preferred embodiment, the photocurable composition further comprises a coinitiators (c), also referred to as accelerators. Suitable examples of accelerators/coinitiators (c) are alcohols, thiols, thioethers, amines or ethers that have an available hydrogen, bonded to a carbon adjacent to the heteroatom, disulfides and phosphines, e.g. as described in EP 438 123 and GB 2 180 358.

Suitable examples of amine accelerators/co-initiators include, but are not limited to, aliphatic, cycloaliphatic, aromatic, aryl-aliphatic, heterocyclic, oligomeric or polymeric amines. They can be primary, secondary or tertiary amines, for example butyl amine, dibutyl amine, tributyl amine, cyclohexyl amine, benzyldimethyl amine, di-cyclohexyl amine, N-phenyl glycine, triethyl amine, phenyl-diethanol amine, triethanolamine, piperidine, piperazine, morpholine, pyridine, quinoline, esters of dimethylamino benzoic acid, Michler's ketone (4,4'-bis-dimethyl aminobenzophenone) and derivatives thereof.

As the amine accelerators/co-initiators, an amine-modified acrylate compound can be used; examples of such amine-modified acrylate include acrylates modified by reaction with a primary or secondary amine that are described in US 3,844,916, EP 280222, US 5,482,649 or US 5,734,002. Preferably said accelerator and/or coinitiator is an amine, preferably a tertiary amine.

Multifunctional amine and polymeric amine derivatives are also suitable as co-initiators some examples are Omnipol^{®} ASA from IGM Resins B.V., Genopol^{®} AB-2 from Rahn A.G., Speedcure^{®} 7040 from Lambson Limited or those described in US2013/0012611.

The photocurable compositions of the present invention can also comprise one or more of the following components: (d) photosensitizers and/or (e) further photoinitiators and/or (f) conventional additives, in addition to compounds (a), (b) and, when present, (c).

The photocurable compositions of the present invention can also be formulated in compositions further comprising water and/or solvents, such as organic solvents.

Photosensitizers (d) can be present in an amount comprised between 0.01 and 15% by weight, based on the total content of the composition, preferably between 0.01 and 10% by weight.

Examples of photosensitizers are those commonly used in the art, aromatic carbonyl compounds, e.g. benzophenones, thioxanthones, anthraquinones, coumarins and 3-acylcoumarin derivatives, terphenyls, styryl ketones, and 3-(aroylmethylene)-thiazolines, camphorquinones and also eosin, rhodamine and erythrosine dyes.

Examples of thioxanthones are thioxanthone, 2-isopropyl thioxanthone, 2-chloro thioxanthone, 2-dodecyl thioxanthone, 2,4-diethyl thioxanthone, 2,4-dimethyl thioxanthone, 1-methoxycarbonyl thioxanthone, 2-ethoxycarbonyl thioxanthone, 3-(2-methoxyethoxycarbonyl) thioxanthone, 4-butoxycarbonyl thioxanthone, 3-butoxycarbonyl-7-methyl thioxanthone, 1-cyano-3-chloro thioxanthone, 1-ethoxycarbonyl-3-chloro thioxanthone, 1-ethoxycarbonyl-3-ethoxy thioxanthone, 1-ethoxycarbonyl-3-amino thioxanthone, 1-ethoxycarbonyl-3-phenylsulfuryl thioxanthone, 3,4-di[2-(2-methoxyethoxy)ethoxycarbonyl] thioxanthone, 1-ethoxycarbonyl-3-(1-methyl-1-morpholinoethyl) thioxanthone, 2-methyl-6-dimethoxymethyl thioxanthone, 2-methyl-6-(1,1-dimethoxybenzyl) thioxanthone, 2-morpholinomethyl thioxanthone, 2-methyl-6-morpholinomethyl thioxanthone, N-allylthioxanthone-3,4-dicarboximide, N-octylthioxanthone-3,4-dicarboximide, N-(1,1,3,3-tetramethylbutyl)-thioxanthone-3,4-dicarboximide, 1-phenoxy thioxanthone, 6-ethoxycarbonyl-1-2-methoxythioxanthone, 6-ethoxycarbonyl-2-methylthioxanthone, thioxanthone-2-polyethylene glycol ester, 2-hydroxy-3-(3,4-dimethyl-9-oxo-9H-thioxanthon-2-yloxy)-N,N,N-trimethyl-1-propanaminium chloride, or those described in the patent application PCT/EP2011/069514, such as n-dodecyl-7-methyl-thioxanthone-3-carboxylate and N,N-disobutyl-7-methyl-thioxanthone-3-carbamide. Also suitable are polymeric thioxanthone derivatives (e.g. Omnipol^{®} TX from IGM Resins B.V., Genopol^{®} TX-1 from Rahn A.G., Speedcure^{®} 7010 from Lambson Limited).

Example of benzophenones are benzophenone, 4-phenyl benzophenone, 4-methoxy benzophenone, 4,4'-dimethoxybenzophenone, 4,4'-dimethyl benzophenone, 4,4'-dichloro benzophenone, 4,4'-dimethylamino benzophenone, 4,4'-diethylamino benzophenone, 4-methyl benzophenone, 2,4,6-trimethyl benzophenone, 4-(4-methylthiophenyl) benzophenone, 3,3'-dimethyl-4-methoxy benzophenone, methyl 2-benzoyl benzoate, 4-(2-hydroxyethylthio) benzophenone, 4-(4-tolylthio) benzophenone, 4-benzoyl-N,N,N-trimethylbenzene methanaminium chloride, 2-hydroxy-3-(4-benzoylphenoxy)-N,N,N-trimethyl-1-propanaminium chloride monohydrate, 4-(13-acryloyl-1,4,7,10,13-pentaoxatridecyl) benzophenone, 4-benzoyl-N,N-dimethyl-N-[2-(1-oxo-2-propenyl)oxylethyl-benzene methanaminium chloride, or those described in US9938231 (e.g. Omnirad^{®} 991 from IGM Resins B.V.).

Also suitable are polymeric benzophenone derivatives (e.g. Omnipol^{®} BP, Omnipol^{®} 2702 and Omnipol^{®} 682 all from IGM Resins B.V., Genopol^{®} BP-2 from Rahn A.G. and Speedcure^{®} 7005 from Lambson Limited). Examples of 3-acylcoumarin derivatives are 3-benzoyl coumarin, 3-benzoyl-7-methoxy coumarin, 3-benzoyl-5,7-di(propoxy) coumarin, 3-benzoyl-6,8-dichloro coumarin, 3-benzoyl-6-chloro coumarin, 3,3'-carbonyl-bis[5,7-di(propoxy) coumarin], 3,3'-carbonyl-bis(7-methoxy coumarin), 3,3'-carbonyl-bis(7-diethylamino coumarin), 3-isobutyroyl coumarin, 3-benzoyl-5,7-dimethoxy coumarin, 3-benzoyl-5,7-diethoxy coumarin, 3-benzoyl-5,7-dibutoxy coumarin, 3-benzoyl-5,7-di(methoxyethoxy) coumarin, 3-benzoyl-5,7-di(allyloxy) coumarin, 3-benzoyl-7-dimethylamino coumarin, 3-benzoyl-7-diethylamino coumarin, 3-isobutyroyl-1,7-dimethylamino coumarin, 5,7-dimethoxy-3-(1-benzoyl) coumarin, 5,7-dimethoxy-3(1-benzoyl)-coumarin, 3-benzoylbenzo [f]coumarin, 7-diethylamino-3-thienoyl coumarin, 3-(4-cyanobenzoyl)-5,7-dimethoxy coumarin, or those described in EP2909243 and WO2017216699.

Examples of 3-(aroylmethylene) thiazolines are 3-methy-1,2-benzoylmethylene-β-benzo thiazoline, 3-methyl-2-benzoylmethylene-benzo thiazoline, 3-ethyl-2-propionylmethylene-β-benzo thiazoline. Examples of other aromatic carbonyl compounds are acetophenone, 3-methoxyacetophenone, 4-phenylacetophenone, benzil, such as that described in WO 2013/164394, 2-acetylnaphthalene, 2-naphthaldehyde, 9,10-anthraquinone, 9-fluorenone, dibenzosuberone, xanthone, 2,5-bis(4-diethylaminobenzylidene) cyclopentanone, α-(para-dimethylamino benzylidene), ketones, such as 2-(4-dimethylamino-benzylidene)-indan-1-one or 3-(4-dimethylaminophenyl)-1-indan-5-yl-propenone, 3-phenylthiophthalimide, N-methyl-3,5-di(ethylthio) phthalimide.

Particularly preferred are thioxanthones, coumarins and 3-acylcoumarins. It was observed that the above components (d) increase the activity of photoinitiators (b) without shortening the shelf life of the compositions. Moreover, such compositions have the special advantage that an appropriate choice of the photosensitizer (d) allows the spectral sensitivity of photoinitioator (b) to be shifted to any desired wavelength region. The skilled in the art is able to select the suitable photosensitizer (d) to make the photoinitiator(s) (b) work at any desired wavelength region.

The further possible photoinitiators (e) can be present in an amount comprised between 0.5 and 15 % by weight, of the total content of the composition, preferably between 1 and 10% by weight of the composition. Examples of other suitable photoinitiators (e) are camphorquinone, benzophenone, benzophenone derivatives, acetophenone, acetophenone derivatives, dialkoxyacetophenones, α-hydroxyketones, α-aminoketones, 4-aroyl-1,3-dioxolanes, benzoin alkyl ethers and benzil ketals, e.g. benzil dimethyl ketal, ketosulfones, e.g 1-[4-[(4-benzoyl-phenyl)-thio]-phenyl]-2-methyl-2-[(4-methyl-phenyl)-sulfonyl]-propan-1-one (Esacure^{®} 1001, from IGM Resins B.V.), 3-ketocoumarins, for example as described in EP2909243 and WO2017216699, phenylglyoxylates and derivatives thereof, dimeric phenyl glyoxylates, peresters, e.g. benzophenonetetracarboxylic acid peresters, for example as described in EP 126 541, acylphosphine photoinitiators (which can be selected from mono-acylphosphine oxides, bis-acylphosphine oxides, tris-acylphosphine oxides and multifunctional mono- or bisacylphosphine oxides), halomethyltriazines, hexaaryl bisimidazole/coinitiator systems, e.g. ortho-chlorohexaphenylbisimidazole in combination with 2-mercaptobenzothiazole, ferrocenium compounds or titanocenes, for example dicyclopentadienyl-bis(2,6-difluoro-3-pyrrolo-phenyl)titanium, O-acyloxime ester photoinitiators.

Examples of α-hydroxyketones and α-aminoketones are 1-hydroxy cyclohexylphenyl ketone, 2-hydroxy-2-methyl-1-phenyl-propane-1-one, 1-[4-(2-hydroxyethoxy)phenyl]-2-hydroxy-2-methyl-1-propane-1-one, 2-hydroxy-1-{4-[4-(2-hydroxy-2-methyl-propionyl)-benzyl]phenyl}-2-methyl-propane-1-one), 2-hydroxy-1-{4-[4-(2-hydroxy-2-methylpropionyl)-phenoxy]-phenyl}-2-methyl-propan-1-one, 2-methyl-1-(4-methylthiophenyl)-2-morpholinopropane-1-one), 2-benzyl-2-dimethylamino-1-(4-morpholinophenyl)-butane-1-one, and (2-(dimethylamino)-2-[(4-methylphenyl)methyl]-1-[4-(4-morpholinyl) phenyl]-1-butanone).

Examples of O-acyloxime ester photoinitiators are 1,2-octanedione,1-[4-(phenylthio)phenyl]-2-(O-benzoyloxime), ethanone 1-[9-ethyl-6-(2-methylbenzoyl)-9H-carbazole-3-yl] 1-(O-acetyloxime) or those described in GB 2339571.

Examples of acylphosphine photoinitiators include, but are not limited to, bis(2,4,6-trimethylbenzoyl)phenylphosphine oxide, bis(2,6-dimethoxybenzoyl)-2,4,4-trimethylpentyl phosphine oxide, bis(2,4,6-trimethylbenzoyl)-(2,4-dipentyloxyphenyl), 2,4,6-trimethylbenzoyl-diphenyl phosphine oxide and ethyl (2,4,6-trimethylbenzoyl) phenylphosphinate, Phenyl(2,4,6-trimethylbenzoyl)phosphinic acid, glycerol ethoxylated trimester (Omnipol^{®} TP from IGM Resins B.V.). Examples of the halomethyltriazines based photoinitiators are 2-[2-(4-methoxy-phenyl)-vinyl]-4,6-bis-trichloromethyl [1,3,5]triazine, 2-(4-methoxy-phenyl)-4,6-bis-trichloromethyl [1,3,5]triazine, 2-(3,4-dimethoxyphenyl)-4,6-bis-trichloromethyl [1,3,5]triazine, 2-methyl-4,6-bis-trichloromethyl [1,3,5] triazine.

Cationic photoinitiators can be also used as the further photoinitiators (e), when the photocurable compositions according to the invention are used in hybrid systems (which in this connection mean mixtures of free- radically and cationically curing systems). Examples of suitable cationic photoinitiators are aromatic sulfonium, phosphonium or iodonium salts, as described e.g. in US4,950,581, or cyclopentadienylarene-iron(II) complex salts, e.g. (η⁶-isopropylbenzene)(η⁵-cyclopentadienyl) iron(II) hexafluorophosphate or photolatent acids based on oximes, as described, for example, in GB 2 348 644, US4,450,598, US4,136,055, WO 00/10972 and WO 00/26219.

The photocuring composition according to the invention may also comprise conventional additives, from 0 to 10% based on the total content of the composition. Additives (f) can be, for example, thermal initiators, binders, stabilizers, and mixture thereof.

The choice of additives is governed by the field of use in question and the properties desired for that field. The additives (f) described above are known in the art and are accordingly used in the amounts conventionally used in the art.

For instance, especially in the case of pigmented compositions, the composition may also comprise, as additional additive (f), a thermal initiator, a compound that forms free radicals when heated, e.g. an azo compounds, such as 2,2'-azobis(4-methoxy-2,4-dimethylvaleronitrile), a triazene, diazosulfide, pentazadiene or a peroxy compound, for example a hydroperoxide or peroxycarbonate, e.g. tert-butyl hydroperoxide, as described e.g. in EP 245 639.

Binders may also be added to the photocurable composition of the invention. The addition of binders is particularly advantageous when the photocurable compounds are liquid or viscous substances. The amount of binder may be, for example, from 5 to 60% by weight, preferably from 10 to 50% by weight, based on the total content of the composition, excluding possible water and solvents. The choice of binder is made in accordance with the field of use and the properties required therefor, such as developability in aqueous and organic solvent systems, adhesion to substrates and sensitivity to oxygen.

Suitable binders are, for example, polymers having a weight average molecular weight (Mw) of approximately from 5,000 Da to 2,000,000 Da, preferably from 10,000 Da to 1,000,000 Da. Illustrative examples are: homo- and copolymers of acrylates and methacrylates, e.g. copolymers of methyl methacrylate/ethyl acrylate/methacrylic acid, poly(methacrylic acid alkyl esters), poly(acrylic acid alkyl esters); cellulose esters and ethers, such as cellulose acetate, cellulose acetate butyrate, methylcellulose, ethylcellulose, polyvinylbutyral, polyvinylformal, cyclised rubber, polyethers such as polyethylene oxide, polypropylene oxide, polytetrahydrofuran, polystyrene, polycarbonates, polyurethanes, chlorinated polyolefins, e.g. polyvinyl chloride, co-polymers of vinyl chloride/vinylidene chloride, co-polymers of vinylidene chloride with acrylonitrile, methyl methacrylate and vinyl acetate, polyvinyl acetate, co-poly (ethylene/vinyl acetate), polymers such as polycaprolactam and poly(hexamethylene adipamide), polyesters such as poly(ethylene glycol terephthalate) and poly(hexamethylene glycol succinate).

Suitable stabilizers are, for example, thermal inhibitors, such as hydroquinone, hydroquinone derivatives, p-methoxyphenol, β-benzol or sterically hindered phenols, e.g. 2,6-di(tert-butyl)-p-cresol, which prevent premature polymerization. In order to increase dark storage stability it is possible to use, for example, copper compounds, such as copper naphthenate, stearate or octoate, phosphorus compounds, for example triphenylphosphine, tributylphosphine, triethyl phosphite, triphenyl phosphite or tribenzyl phosphite, quaternary ammonium compounds, e.g. tetramethylammonium chloride or trimethylbenzylammonium chloride, or hydroxylamine derivatives, e.g. N,N-diethylhydroxylamine. For the purpose of excluding atmospheric oxygen during polymerization it is possible to add paraffin or similar wax-like substances which, being insoluble in the polymer, migrate to the surface at the beginning of the polymerization and form a transparent surface layer which prevents air from entering.

It is also possible to add a light stabilizer, such as UV absorbers, e.g. hydroxyphenylbenzotriazole, hydroxyphenylbenzophenone, oxalic acid amide or hydroxyphenyl-s-triazine type. Such components can be used on their own or in the form of mixtures, with or without the use of sterically hindered amines (HALS).

The photocurable compositions according to the invention may also comprise, as further additives (f), photoreducible dyes, e.g. a xanthene, benzoxanthene, benzothioxanthene, thiazine, pyronin, porphyrin or acridine dye, and/or radiation cleavable trihalomethyl compounds. These compounds are described, for example, in EP445624.

Further customary additives (f) are, depending upon the intended use, optical brighteners, fillers, pigments, both white and colored pigments, colorants, antistatics, wetting agents or flow improvers. Additives conventionally used in the art, e.g. antistatics, flow improvers and adhesion enhancers, can also be used.

In addition to the above components, other components may be present in the composition of the invention.

It is also possible for chain-transfer reagents conventionally used in the art to be added to the compositions according to the invention. Examples are mercaptans, amines and benzothiazole.

The composition of the invention may also comprise colorants and/or colored pigments. Depending upon the intended use, both inorganic and organic pigments may be used. Such additives are well known to the person skilled in the art; some examples are carbon black, iron oxides, such as iron oxide yellow, iron oxide red, chromium yellow, chromium green, nickel titanium yellow, ultramarine blue, cobalt blue, bismuth vanadate, cadmium yellow and cadmium red. Examples of organic pigments are mono- or bis-azo pigments, and also metal complexes thereof, phthalocyanine pigments, polycyclic pigments, e.g. perylene, anthraquinone, thioindigo, quinacridone or triphenylmethane pigments, and also diketo-pyrrolo-pyrrole, isoindolinone, e.g. tetrachloroisoindolinone, isoindoline, dioxazine, benzimidazolone and quinophthalone pigments. The pigments may be used in the formulations on their own or in admixture.

Depending upon the intended use, the pigments can be added to the formulations in amounts conventionally used in the art, for example in an amount from 0.1 to 30% by weight or from 10 to 25% by weight, based on the total weight of the composition.

The composition may also comprise, for example, organic colorants of an extremely wide variety of classes. Examples are azo dyes, methine dyes, anthraquinone dyes and metal complex dyes. Usual concentrations are, for example, from 0.1 to 20% wt, especially from 1 to 5% wt, based on the total weight of the composition.

The photocurable compositions of the invention may comprise water.

The photocurable compositions of the invention are suitable for various purposes, for example as a printing ink, such as screen printing inks, flexographic printing inks, offset printing inks and inkjet printing inks, as clearcoats, as colored coats, for example for wood or metal, as powder coatings, as coating materials inter alia for paper, wood, metal or plastics, as daylight-curable paints for marking structures and roads, for photographic reproduction processes, for holographic recording materials, for image-recording processes or in the production of printing plates that can be developed using organic solvents or using aqueous-alkaline media, for the production of masks for screen printing, as dental filling compounds, as adhesives, as pressure-sensitive adhesives, as laminating resins, as photoresists, e.g. galvanoresists, as etch resists or permanent resists, both liquid and dry films, as photostructurable dielectrics, and as solder masks for electronic circuits, as resists in the production of color filters for any type of display screen or in the creation of structures during the manufacture of plasma displays and electroluminescent displays, in the production of optical switches, optical gratings (interference gratings), in the manufacture of three-dimensional articles by bulk curing (UV curing in transparent moulds) or according to the stereolithography process, as described, for example, in US4,575,330, in the manufacture of composite materials (e.g. styrene polyesters which may include glass fibers and/or other fibers and other adjuvants) and other methods of printing in three dimensions well-known to one skilled in the art, in the coating or sealing of electronic components or as coatings for optical fibers.

The photocurable compositions of the invention are also suitable for the production of optical lenses, e.g. contact lenses or Fresnel lenses, in the manufacture of medical apparatus, aids or implants, in dry film paints. The photocurable compositions of the invention are also suitable for the preparation of gels having thermotropic properties. Such gels are described for example in DE 19700064 and EP 678534.

An article comprising a compound of formula (I), or comprising a photocurable composition of the invention is also described but not part of the invention.

The compounds and compositions according to the invention may also be used as free-radical photoinitiators or photoinitiating systems for radiation-curable powder coatings.

The photocurable compositions according to the invention are suitable, for example, as coating materials for all kinds of substrate, for example wood, textiles, paper, ceramics, glass, plastics, such as polyesters, polyethylene terephthalate, polyolefins and cellulose acetate, especially in the form of films, and also metals, such as Al, Cu, Ni, Fe, Zn, Mg or Co and GaAs, Si or SiO₂, to which a protective layer is to be applied or an image is to be applied e.g. by imagewise exposure.

A large number of the most varied kinds of light source may be used in the process according to the invention, the light source emitting at wavelengths from approximately 200 nm to approximately 800 nm. Both point sources and planiform radiators (lamp carpets) are suitable.

Examples are: carbon arc lamps, xenon arc lamps, medium pressure, high pressure and low pressure mercury arc radiators, doped, where appropriate, with metal halides (metal halide lamps), microwave-excited metal vapour lamps, excimer lamps, superactinic fluorescent tubes, fluorescent lamps, argon incandescent lamps, flash lamps, photographic floodlight lamps, light-emitting diodes (LED), electron beams, X-rays and lasers.

According to one embodiment, said light source comprises UV light in at least one of the UVA, UVB and UVC ranges.

According to a preferred embodiment, said light source is a LED source, particularly preferred are LED light source emitting at wavelengths comprised between 365 nm and 420 nm, more preferably at 365 nm, 385 nm and 395 nm.

According to the invention the distance between the lamp and the substrate to be exposed may vary according to the intended use and the type and strength of the lamp, e.g. from 0.1 cm to 150 cm, preferably from 1 cm to 50 cm.

Said photopolymerizable composition may also be applied over a substrate already comprising a coated or printed layer. Said photopolymerizable composition may, after photopolymerization with said light source, be overprinted or overcoated with one or more compositions suitable for printing or coating.

The article obtained by applying said photopolymerizable composition to said substrate by said means of coating or printing, and photopolymerizing by said light source, with or without further elaboration of the article by further coating or printing, is also described, but not part of the claimed invention.

As said, we surprisingly found that compound of formula (I) has a very high reactivity under UVA, UVB and UVC wavelength maintaining a low post cure yellowing compared to those of the prior art. The new compound showed its great improvement in surface curing under Hg lamps both alone or in combination with a coinitiator.

The invention is illustrated in detail below by the following examples, which are illustrative and not limiting.

### EXPERIMENTAL SECTION

In all the present specification, in case of inconsistences between a chemical name and a chemical formula, the latter prevails.

¹H NMR spectra were recorded with a Bruker Ascend 300 MHz NMR Spectrometer.

### Example 1

### Synthesis of

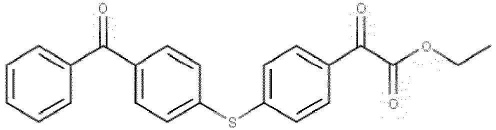

To an ice-cooled mixture of 10.00 g (53.686 mmoles) of Diphenyl sulfide and 7.92 g (56.342 mmoles) of Benzoyl chloride in 120 mL of dichloromethane, 7.87 g (59.022 mmoles) of anhydrous aluminum chloride were cautiously added in portions. After stirring for 1.5 hours at room temperature, the reaction was cooled again and 9.53 g (69.802 mmoles) of Ethyl chlorooxoacetate and 10.88 g (81.596 mmoles) of anhydrous aluminum chloride were cautiously added in sequence. After stirring for 1.5 hours at room temperature, the reaction was cooled again and additional 1.47 g (10.767 mmoles) of Ethyl chlorooxoacetate and 1.65 g (12.374 mmoles) of anhydrous aluminum chloride were cautiously added in sequence. Then the reaction was stirred at room temperature for further 1.5 hours and poured into 400 mL of ice/water. The mixture was extracted with dichloromethane and the organic layer was separated, washed again with water and brine, dried over anhydrous sodium sulfate, filtered and the solvent removed by distillation under vacuum obtaining the crude. The crude product was purified by crystallization from ethanol obtaining 15.70 g of an off-white solid (yield 75%).

¹H-NMR (DMSO-d₆, δ ppm): 1.33 (t, 3H), 4.42 (q, 2H), 7.51 (d, 2H), 7.54-7.74 (m, 5H), 7.76-7.82 (m, 4H), 7.97 (d, 2H).

### Comparative Tests

The photoinitiator (PI) of the invention was compared with Compound G-1A-32 of EP3597668 page 25 (COMP-1) and Compound C24 of KR20200160890 (COMP-2).

### Example 2

### Tack-free in clear formulation

The photopolymerizable compositions for the test were prepared by dissolving the photoinitiators at a concentration of 5 % by wt each in a solution of Photomer 6628 (aliphatic urethane hexaacrylate) 50%, Photomer 4335 (PETIA) 15%, Photomer 4600 (DPHA) 15%, Photomer 4172 (PPTTA) 20%.

The photopolymerizable composition was stirred for 1 hours at 60°C then spread with a thickness of 50 microns on a varnished cardboard using a bar-coater. Therefore, is photopolymerized using a Mercury lamp (120 W/cm) at a distance of 8 cm.

The results are expressed in meters per minutes as the maximum speed at which the tack-free is reached (Table 3).

**Table 3**

| Photoinitiator | Tack-free (m/min) |
|---|---|
| EXAMPLE 1 | 23 |
| COMP-1* | 15 |
| COMP-2* | 19 |

| | |
|---|---|
| *Comparative | |

This test confirms that compound of formula (I) is more reactive than the two comparative PIs of the prior art.

### Example 3

### Yellow Index

The yellowing was measured as Yellow Index (YI) E313 illuminant D65/2 using a TECHKON SpectroDens Premium. The formulation prepared in example 2 was spread with a thickness of 50 microns on a varnished cardboard using a bar-coater and cured with a Mercury Lamp (120 W/cm) at the maximum speed at which the tack-free is reached. Then, the yellowing as Yellow Index (YI) is measured, the results are shown in Table 4.

**Table 4**

| Photoinitiator | Yellow Index |
|---|---|
| EXAMPLE 1 | 7.02 |
| COMP-1* | 8.93 |
| COMP-2* | 8.57 |

| | |
|---|---|
| * Comparative | |

This test shows that compound of formula (I) is also lower yellowing compared to the two comparative PIs of the prior art.

### Example 4

Tack-free in clear formulation in presence of a coinitiator The photopolymerizable compositions for the test were prepared by dissolving the photoinitiators at a concentration of 5 % by wt each in a solution of Photomer 6628 (aliphatic urethane hexaacrylate) 50%, Photomer 4335 (PETIA) 15%, Photomer 4600 (DPHA) 15%, Photomer 4172 (PPTTA) 20%. The coinitiator (Esacure A198) was added at the compositions in the same amount (5% by wt).

The photopolymerizable composition was stirred for 1 hours at 60°C then spread with a thickness of 50 microns on a varnished cardboard using a bar-coater. Therefore, is photopolymerized using a Mercury lamp (120 W/cm) at a distance of 8 cm.

The results are expressed in meters per minutes as the maximum speed at which the tack-free is reached (Table 3).

**Table 3**

| Photoinitiator | Tack-free (m/min) |
|---|---|
| EXAMPLE 1 | 98 |
| COMP-1* | 78 |
| COMP-2* | 89 |

| | |
|---|---|
| *Comparative | |

All these tests confirm that compound of formula (I) is more reactive than that of the prior art and surprising the good reactivity does not go with a high yellowing after curing.

## Claims

1. A compound of formula (I)

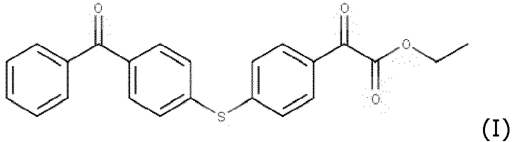

2. A photopolymerizable composition comprising:
a) from 50 to 99.9% by weight, based on the total content of the composition of at least one ethylenically unsaturated compound;
b) from 0.1 to 35%, preferably from 0.2 to 15% by weight, based on the total content of the composition, of the compound of formula (I), as defined in claim 1; and
c) from 0 to 20% by weight, preferably from 0.2 to 15% by weight, based on the total content of the composition, of an accelerator and/or of a coinitiator.

3. The photocurable composition of claim 2, **characterized in that** it comprises:
a) from 70 to 98.9% by weight, based on the total content of the composition of at least one ethylenically unsaturated compound;
b) from 0.1 to 20%, and more preferably from 0.2 to 15% by weight, based on the total content of the composition, of the compound of formula (I), as defined in claim 1; and
c) from 0 to 15%, and more preferably from 0.2 to 15% by weight, based on the total content of the composition, of an accelerator and/or of a coinitiator.

4. The photocurable composition of claim 2 or 3, **characterized in that** said accelerator and/or coinitiator is an amine, preferably a tertiary amine.

5. The photocurable composition of any one of claims 2 to 4, further comprising one or more of the following components:
d) from 0.01 to 15% by weight based on the total content of the composition, of one or more photosensitizer; and/or
e) from 0.5 to 15% by weight based on the total content of the composition, one or more further photoinitiators.

6. A process for photocuring photopolymerizable compositions, coatings, adhesives and inks, which process comprises:
(i) providing a photopolymerizable composition of any one of claims 2 to 5;
(ii) coating or printing said photopolymerizable composition onto a substrate; and
(iii) photopolymerizing said coated or printed composition on said substrate with a light source.

7. A process for three-dimensional printing which comprises providing a photopolymerizable composition of any one of claims 2 to 5 and photocuring said composition with a light source.

8. The process of claims 6 or 7, **characterized in that** said light source comprises UV light in at least one of the UVA, UVB and UVC ranges.

9. The process of any one of claims 6 to 8, **characterized in that** said light source is
- a Hg lamp; or
- a LED source emitting in the range from 350 to 420 nm.

10. The process of any one of claims 6 to 9 further comprising the step of applying said photopolymerizable composition to a substrate prior to photopolymerizing it.

## Patentansprüche

1. Eine Verbindung der Formel (I)

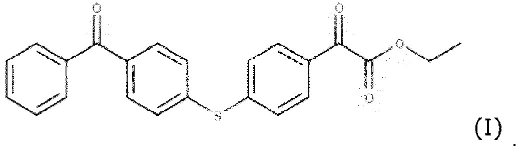

2. Eine photopolymerisierbare Zusammensetzung, umfassend:
a) 50 bis 99,9 Gew.-%, bezogen auf den Gesamtgehalt der Zusammensetzung, mindestens einer ethylenisch ungesättigten Verbindung;
b) 0,1 bis 35 Gew.-%, vorzugsweise 0,2 bis 15 Gew.-%, bezogen auf den Gesamtgehalt der Zusammensetzung, mindestens einer Verbindung der Formel (I), wie in Anspruch 1 definiert; und
c) 0 bis 20 Gew.-%, vorzugsweise 0,2 bis 15 Gew.-%, bezogen auf den Gesamtgehalt der Zusammensetzung, eines Beschleunigers und/oder eines Coinitiators.

3. Die photohärtbare Zusammensetzung gemäß Anspruch 2, **dadurch gekennzeichnet, dass** sie Folgendes umfasst:
a) 70 bis 98,9 Gew.-%, bezogen auf den Gesamtgehalt der Zusammensetzung, mindestens einer ethylenisch ungesättigten Verbindung;
b) 0,1 bis 20 Gew.-%, und bevorzugter 0,2 bis 15 Gew.-%, bezogen auf den Gesamtgehalt der Zusammensetzung, mindestens einer Verbindung der Formel (I), wie in Anspruch 1 definiert; und
c) 0 bis 15 Gew.-%, und bevorzugter 0,2 bis 15 Gew.-%, bezogen auf den Gesamtgehalt der Zusammensetzung, eines Beschleunigers und/oder eines Coinitiators.

4. Die lichthärtbare Zusammensetzung nach Anspruch 2 oder 3, **dadurch gekennzeichnet, dass** der Beschleuniger und/oder Coinitiator ein Amin, vorzugsweise ein tertiäres Amin ist.

5. Die photohärtbare Zusammensetzung nach einem der Ansprüche 2 bis 4, die außerdem eine oder mehrere der folgenden Komponenten enthält:
d) 0,01 bis 15 Gew.-%, bezogen auf den Gesamtgehalt der Zusammensetzung, eines oder mehrerer Photosensibilisatoren; und/oder
e) 0,5 bis 15 Gew.-%, bezogen auf den Gesamtgehalt der Zusammensetzung, eines oder mehrerer weiterer Photoinitiatoren.

6. Ein Verfahren zum Photohärten von photopolymerisierbaren Zusammensetzungen, Beschichtungen, Klebstoffen und Tinten, welches umfasst:
(i) Bereitstellen einer photopolymerisierbaren Zusammensetzung wie in einem der Ansprüche 2 bis 5 definiert;
(ii) Beschichten oder Drucken der photopolymerisierbaren Zusammensetzung auf ein Substrat; und
(iii) Photopolymerisieren der beschichteten oder gedruckten Zusammensetzung auf dem Substrat mit einer Lichtquelle.

7. Ein Verfahren zum dreidimensionalen Drucken, das das Bereitstellen einer photopolymerisierbaren Zusammensetzung gemäß einem der Ansprüche 2 bis 5 und das Photohärten dieser Zusammensetzung mit einer Lichtquelle umfasst.

8. Das Verfahren nach Anspruch 6 oder 7, **dadurch gekennzeichnet, dass** die Lichtquelle UV-Licht in mindestens einem der UVA-, UVB- und UVC-Bereiche umfasst.

9. Das Verfahren gemäß einem der Ansprüche 6 bis 8, **dadurch gekennzeichnet, dass** die Lichtquelle
- eine Hg-Lampe; oder
- eine LED-Quelle, die im Bereich von 350 bis 420 nm emittiert, ist.

10. Das Verfahren gemäß einem der Ansprüche 6 bis 9, das außerdem den Schritt des Aufbringens der photopolymerisierbaren Zusammensetzung auf ein Substrat vor ihrer Photopolymerisierung umfasst.

## Revendications

1. Composé de formule (I)

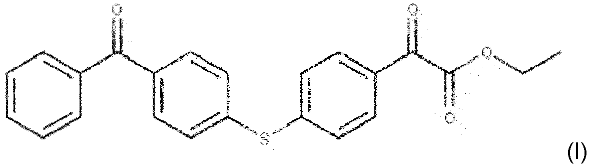

2. Composition photopolymérisable comprenant
a) de 50 à 99,9 % en poids, par rapport à la teneur totale de la composition, d'au moins un composé éthyléniquement insaturé ;
b) de 0,1 à 35 %, de préférence de 0,2 à 15 % en poids, par rapport à la teneur totale de la composition, du composé de formule (I), tel que défini dans la revendication 1 ; et
c) de 0 à 20 % en poids, de préférence de 0,2 à 15 % en poids, par rapport à la teneur totale de la composition, d'un accélérateur et/ou d'un coinitiateur.

3. Composition photodurcissable de la revendication 2, **caractérisée en ce qu'**elle comprend :
a) de 70 à 98,9 % en poids, par rapport à la teneur totale de la composition, d'au moins un composé éthyléniquement insaturé ;
b) de 0,1 à 20 %, et plus préférentiellement de 0,2 à 15 % en poids, par rapport à la teneur totale de la composition, du composé de formule (I), tel que défini dans la revendication 1 ; et
c) de 0 à 15 %, et de préférence de 0,2 à 15 % en poids, par rapport à la teneur totale de la composition, d'un accélérateur et/ou d'un coinitiateur.

4. Composition photodurcissable de la revendication 2 ou 3, **caractérisée en ce que** ledit accélérateur et/ou coinitiateur est une amine, de préférence une amine tertiaire.

5. Composition photodurcissable de l'une quelconque des revendications 2 à 4, comprenant en outre un ou plusieurs des composants suivants :
d) de 0,01 à 15 % en poids par rapport à la teneur totale de la composition, d'un ou plusieurs photosensibilisateurs ; et/ou
e) de 0,5 à 15 % en poids par rapport à la teneur totale de la composition, un ou plusieurs autres photo-initiateurs.

6. Procédé consistant à photopolymériser des compositions de photopolymérisation, des revêtements, d'adhésifs et d'encres photopolymérisables, comprenant les étapes consistant à :
(i) fournir une composition photopolymérisable selon l'une quelconque des revendications 2 à 5 ;
(ii) revêtir ou imprimer ladite composition photopolymérisable sur un substrat ; et
(iii) photopolymériser ladite composition revêtue ou imprimée sur ledit substrat à l'aide d'une source de lumière.

7. Procédé d'impression tridimensionnelle consistant à fournir une composition photopolymérisable selon l'une quelconque des revendications 2 à 5 et à photopolymériser ladite composition à l'aide d'une source de lumière.

8. Procédé des revendications 6 ou 7, **caractérisé en ce que** ladite source de lumière comprend une lumière UV dans au moins une des gammes UVA, UVB et UVC.

9. Procédé de l'une quelconque des revendications 6 à 8, **caractérisé en ce que** ladite source de lumière est
- une lampe Hg ; ou
- une source LED émettant dans la gamme de 350 à 420 nm.

10. Procédé de l'une quelconque des revendications 6 à 9, comprenant en outre l'étape consistant à appliquer ladite composition photopolymérisable sur un substrat avant de le photopolymériser.
